(19) Europäisches Patentamt
European Patent Office
Office européen des brevets

(11) Veröffentlichungsnummer: **0 463 357 A1**

(12) # EUROPÄISCHE PATENTANMELDUNG

(21) Anmeldenummer: **91108082.8**

(51) Int. Cl.$^5$: **C07C 309/29**, C07C 303/22

(22) Anmeldetag: **18.05.91**

(30) Priorität: **31.05.90 DE 4017568**

(43) Veröffentlichungstag der Anmeldung:
**02.01.92 Patentblatt 92/01**

(84) Benannte Vertragsstaaten:
**CH DE FR GB IT LI**

(71) Anmelder: **BAYER AG**

**W-5090 Leverkusen 1 Bayerwerk(DE)**

(72) Erfinder: **Bader, Axel**
**Heymannstrasse 40**
**W-5090 Leverkusen(DE)**
Erfinder: **Arlt, Dieter, Prof.Dr.**
**Rybniker Strasse 2**
**W-5000 Köln 80(DE)**
Erfinder: **Seng, Florin**
**Am Kattterbach 46**
**W-5060 Bergisch Gladbach(DE)**

(54) **Bisstilbenverbindungen.**

(57) Sulfonsäuren von Bisstyrylbiphenylen, die wertvolle optische Aufheller sind, erhält man in guten Ausbeuten auf einfache Weise, indem man Styrolverbindungen mit 4,4'-Dibrombiphenyl-3,3'-disulfonsäuren oder deren Salzen in Gegenwart von Basen und Palladiumkatalysatoren umsetzt.

EP 0 463 357 A1

Gegenstand der Erfindung sind Bisstilbenverbindungen der Formel

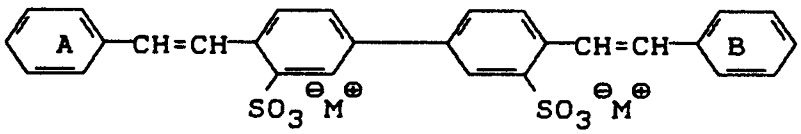

(I)

worin

$M^{\oplus}$ ein Kation bedeutet und

mindestens einer der Ringe A oder B eine Sulfogruppe enthält.

Diese Verbindungen besitzen ein stärkeres Aufhellvermögen als die herkömmlicherweise für synthetische Fasern, insbesondere Zellulose- oder Polyamidfasern verwendeten. Die erfindungsgemäßen Verbindungen können nach einer Vielzahl an sich bekannter Verfahren hergestellt werden.

Ein technisch brauchbares und vorteilhaftes Verfahren besteht darin, daß man eine Verbindung der allgemeinen Formel

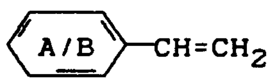

(II)

mit Halogenverbindungen der Formel (III)

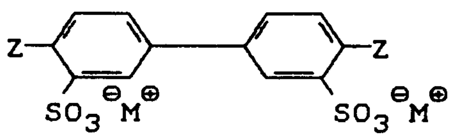

(III)

wobei

Z für Cl, Br oder I steht

und die übrigen Symbole die obengenannte Bedeutung haben, in Gegenwart einer Base und eines Palladium-Komplexes oder einer Kombination von diesen Komplex bildenden Verbindungen umsetzt.

Diese Umsetzung ist für spezielle Olefine im Prinzip bekannt (vergl. J. Amer. Chem. Soc. 96, 1133 (1974) sowie US-Patentschrift 3 922 299).

In Frage kommen z.B. Organophosphinpalladiumkomplexe der Formel

$X_2Pd(PR_3)_2$

wobei

X Halogen, Cyano, Nitro, ($OOC-C_1-C_{12}$-Alkyl)

R Aryl, Alkoxy, Alkyl oder Phenoxy

bedeuten.

Geeignete Verbindungen, die diesen Katalysator bilden, sind Kombinationen aus Palladiumverbindungen und Phosphinen oder Phosphiten.

Beispiele für solche Palladiumverbindungen sind:

$PdCl_2$, $PdBr_2$, $Pd(CN)_2$, $Pd(NO_3)_2$,

$Pd(CH_3COCHCOCH_3)_2$,

Pd(OOC-$C_{1-12}$-Alkyl)$_2$, besonders Palladiumacetat. Es kommen jedoch auch Palladium(O)-Komplexe, wie z.B. Bis-(Dibenzylidenaceton)palladium(O), Bis-(Phenylisonitril)palladium(O) und Tetrakis(triphenylphosphin)-palladium in Frage.

Als Beispiele für die Phosphorverbindungen seien genannt:

Diphenylmethylphosphin, Diphenylmethoxyphosphin, Trimethylphosphin, Triethylphosphin, Tri-n-butylphosphin, Triphenylphosphin, Phenyl-di-n-butoxyphosphin, Tri-o-tolylphosphin und Triphenylphosphit. Bevorzugt sind die Trialkyl- und Triphenylphosphine.

Gut geeignet als Reaktionsmedium sind cyclische und N,N-disubstituierte Amide.

Als Beispiele seien genannt:

N,N-Dimethylformamid, N,N-Diethylformamid, N,N-Di-n-butylformamid, N,N-Diisopentylformamid, N,N-Dimethylacetamid, N,N-Dimethylpropionamid, N-Methyl-N-benzylformamid, N-Ethyl-N-cyclohexylformamid, N-Formylpiperidin, N-Formylpyrrolidin, N-Acetylmorpholin, N-Methylpyrrolidon, N-Ethylpyrrolidon, N-Methylpiperidon und N-Methyl-caprolactam. Bevorzugt verwendet man als Lösungsmittel N,N-Dimethylformamid, N,N-Diethylformamid, N,N-Dimethylacetamid oder N-Methylpyrrolidon. Besonders bevorzugt sind N,N-Dimethylformamid und N-Methylpyrrolidon.

Ebenso ist es möglich, Mischungen dieser Verbindungen mit anderen Lösungsmitteln einzusetzen.

Die Katalysatoren werden im allgemeinen in einer Menge von 0,001 bis 5 Mol-%, bezogen auf die Verbindung (III), verwendet.

Die Reaktions-Temperaturen liegen im allgemeinen zwischen 50 und 200 °C, vorzugsweise bei 90 bis 160 °C.

Geeignete Basen zur Umsetzung von (II) mit (III) sind Alkali- sowie Erdalkalisalze von aliphatischen und aromatischen Carbonsäuren (z.B. Acetate und Benzoate).

Geeignete Kationen $M^{\oplus}$ in den obengenannten Formeln sind Protonen und Alkali- sowie Erdalkaliionen. Bevorzugt sind $Na^{\oplus}$ und $K^{\oplus}$.

Unter "Alkyl" bzw. "Alkoxy" werden im Rahmen dieser Erfindung vorzugsweise Reste mit 1 bis 4 C-Atomen verstanden.

Geeignetes "Halogen" Z und X ist Cl, Br und J, besonders Br für Z und Cl für X.

Geeignete Arylreste sind vor allem Phenylreste, die z.B. durch Cl oder $CH_3$ substituiert sein können.

Die erfindungsgemäßen Verbindungen der allgemeinen Formel (I) haben in Lösung ein hohes Fluoreszenzvermögen und bewirken einen ausgezeichneten Aufhelleffekt, wenn sie auf natürliche und regenerierte Zellulosefasern, Polyvinylalkoholfasern und stickstoffhaltige Fasern, wie Polyamidfasern, Proteinfasern und Polyurethanfasern aufgebracht werden. Sie können auch bei Papier, Pulpen, echtem und synthetischem Leder, Kaseinkunststoffen und Harzen, wie Polyamid-, Polyurethan- und Melaminharzen, sowie bei Lacken, Anstrichen, Seifen, synthetischen Detergentien und Sonnenlotionen angewandt werden.

Die Behandlung mit den erfindungsgemäßen Aufhellern kann in folgender Weise bewirkt werden:

a) in herkömmlicher Weise durch Behandlung in einer wäßrigen Lösung,

b) durch Fixierung in der Wärme, durch Wasserdampfbehandlung oder Säureschock,

c) bei Zellulose- und Polyamidfasern in einer Waschflüssigkeit, die sowohl ein Detergens oder eine Seife als auch eine Verbindung der allgemeinen Formel (I) enthält,

d) zusammen mit einem chemischen Bleichmittel oder durch Zugabe der Verbindung (I) zu einem Bleichbad,

e) wenn Fasern oder Harze in einem Bad behandelt werden, durch Lösen der Verbindung (I) in einem organischen Lösungsmittel und, falls notwendig, Wasser (Anwendung des Färbeverfahrens in einem Lösungsmittel),

f) bei Papier, zum Beispiel durch Zugabe einer Verbindung (I) zum Papierbrei, durch Überziehen der Oberfläche oder durch Schlichten,

g) in Kombination mit anderen fluoreszierenden Aufhellern zur Korrigierung der Farbe und/oder zur Erzielung verschiedener synergistischer Effekte.

Ein Merkmal der erfindungsgemäßen Verbindungen liegt darin, daß sie nicht nur sehr gute Wasch- und Lichtechtheit, sondern auch ausgezeichnete Beständigkeit gegenüber Chlor und Chloriten aufweisen und ein starkes Aufhellvermögen für Zellulose- und Polyamidfasern besitzen. Insbesondere, wenn eine Polyamidfaser in einer Waschflüssigkeit, die sowohl ein Detergens (zum Beispiel ein synthetisches und flüssiges Detergens) oder Seife und die erfindungsgemäße Verbindung enthält, gleichzeitig gewaschen und aufgehellt wird, ist der erzielte Aufhelleffekt wesentlich besser als der mit den bisher bekannten Aufhellern erreichte. Ein anderes Merkmal der erfindungsgemäßen Verbindungen besteht in ihrer starken Affinität zu sowohl Zellulose- als auch Polyamidfasern bei niedrigen und höheren Waschtemperaturen.

Selbst in Mengen von nur 0,001 % des zu behandelnden Materials bewirken diese Verbindungen manchmal einen guten Aufhelleffekt. Im allgemeinen werden diese Verbindungen jedoch in einer Menge

von 0,5 % oder mehr und insbesondere von 0,005 bis 0,2 % verwendet.

Beispiele

Beispiel 1

In 100 ml DMF erhitzt man unter $N_2$ 5,16 g (10 mmol) 4,4'-Dibrombiphenyl-3,3'-disulfonsäuredinatriumsalz, 5,15 g (25 mmol) Styrol-4-sulfonsäurenatriumsalz, 3,3 g (40 mmol) Natriumacetat, 0,045 g (0,2 mmol) Palladium(II)acetat, 122 mg (0,4 mmol) Tri-o-tolylphosphin und 0,035 g Hydrochinon 6 h auf 120 °C. Man dampft zur Trockne ein, nimmt den Rückstand in wenig Wasser auf, filtriert, säuert das Filtrat mit konzentrierter HCl an, saugt ab und trocknet. Man erhält 5,63 g (83 %) der Verbindung der Formel

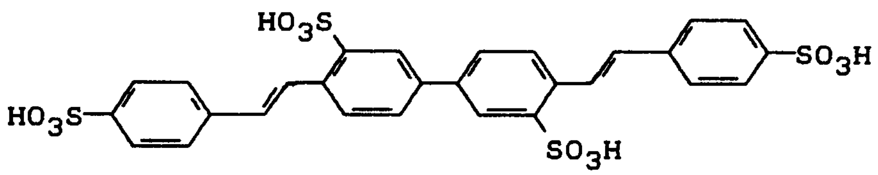

| | |
|---|---|
| $^1$H-NMR (DMSO-$d_6$), $\delta$ = | 7,25 (d,2H), 7,5-7,8 (m,10H), 7,96 (d,2H), 8,2 (d,2H), 8,34 (d,2H) |
| UV (DMF) $\epsilon_{357nm}$ = | 62.200 |

Beispiel 2

Man verfährt wie in Beispiel 1, anstelle von DMF wird jedoch N-Methylpyrrolidon verwendet und das Reaktionsgemisch 3 h auf 150 °C erhitzt. Ausbeute: 5,4 g (80 %).

Beispiel 3

Man verfährt wie in Beispiel 1, anstelle von Styrol-4-sulfonsäurenatriumsalz wird jedoch Styrol-2-sulfonsäurenatriumsalz eingesetzt. Als Produkt erhält man die Verbindung der Formel

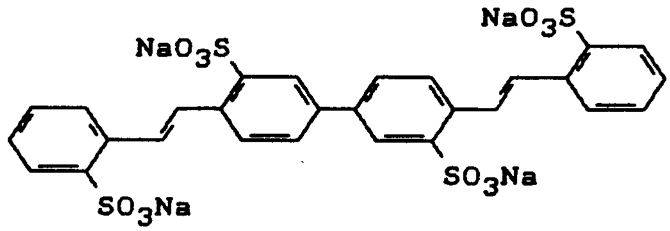

in einer Ausbeute von 5,67 g (74 %), wenn man den Rückstand, der nach Abziehen des DMF verbleibt, in wenig NaCl-Lösung verrührt, das Produkt absaugt und trocknet.

UV (DMF) $\epsilon_{345nm}$ = 56.300

Beispiel 4

Man verfährt wie in Beispiel 3, anstelle von Styrol-2-sulfonsäurenatriumsalz wird jedoch ein 1/1-Gemisch aus Styrol-4-sulfonsäurenatriumsalz und Styrol-2-sulfonsäurenatriumsalz eingesetzt. Man erhält ein Produktgemisch der Formel

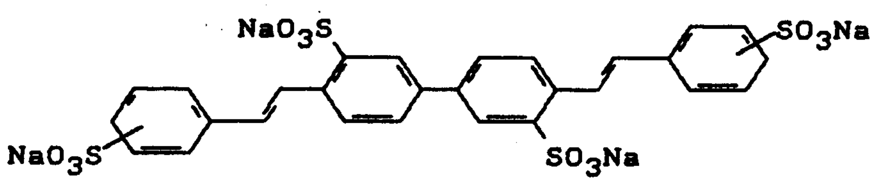

wobei sich die Sulfogruppen in den Styrolresten jeweils entweder in o- oder p-Stellung befinden.

UV (DMF) $\epsilon_{346nm}$ = 58.000

Beispiel 5

Man verfährt wie in Beispiel 3, anstelle von Styrol-2-sulfonsäurenatriumsalz wird jedoch ein 1/1-Gemisch aus Styrol-4-sulfonsäurenatriumsalz und Styrol eingesetzt. Als Produkt erhält man ein Gemisch, dessen Hauptkomponente die Verbindung der Formel

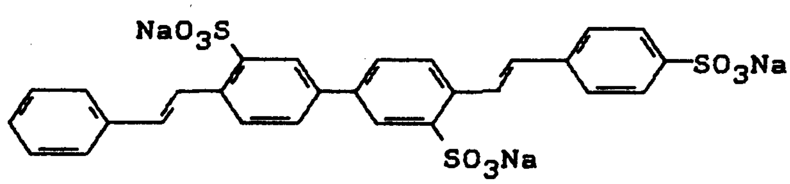

ist.

Ausbeute: 5,37 g

UV (DMF) $\epsilon_{354nm}$ = 58.000

**Patentansprüche**

**1.** Bisstilbenverbindungen der Formel (I)

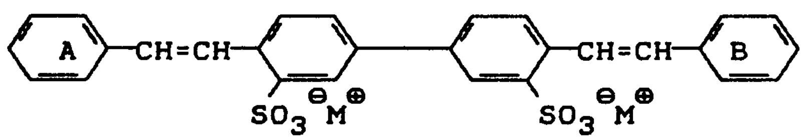

(I)

worin

$M^{\oplus}$ ein Kation bedeutet und

mindestens einer der Ringe A oder B eine Sulfogruppe enthält.

**2.** Bisstilbenverbindungen gemäß Anspruch 1, dadurch gekennzeichnet, daß die Ringe A und B je eine Sulfogruppe enthalten.

**3.** Bisstilbenverbindungen gemäß Anspruch 1 oder 2, dadurch gekennzeichnet, daß sich die Sulfogruppen in ortho- oder para-Stellung befinden.

**4.** Verfahren zur Herstellung von Verbindungen der Formel (I)

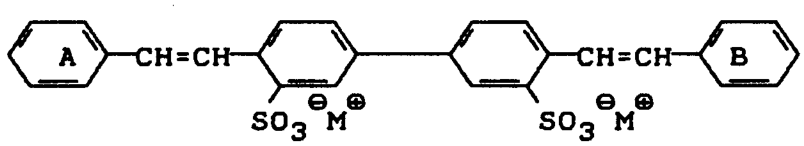

(I)

worin

$M^{\oplus}$ ein Kation bedeutet und

mindestens einer der Ringe A und B eine Sulfogruppe enthält,

dadurch gekennzeichnet, daß man Styrylverbindungen der Formel (II)

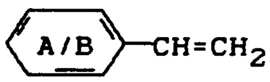

(II)

mit Halogenverbindungen der Formel (III)

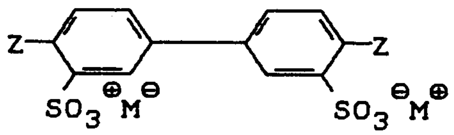

(III)

wobei

Z Cl, Br oder J bedeutet,

und die übrigen Symbole die obengenannte Bedeutung haben, in Gegenwart einer Base und eines Organophosphin-palladium-Komplexes oder einer Kombination von diesen Komplex bildenden Verbindungen umsetzt.

**5.** Verfahren nach Anspruch 4, dadurch gekennzeichnet, daß man die Palladiumkomplexe in situ im Reaktionsmedium aus einem Palladiumsalz und einem Phosphin herstellt.

Europäisches Patentamt

# EUROPÄISCHER RECHERCHENBERICHT

Nummer der Anmeldung

**EP 91 10 8082**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 364 403 (CIBA-GEIGY)<br>* Beispiele; Ansprüche *<br>– – – | 1-5 | C 07 C 309/29<br>C 07 C 303/22 |
| A | CH-A-6 408 99 (CIBA-GEIGY)<br>* Anspruch 7 *<br>– – – | 1-5 | |
| A | EP-A-0 032 483 (CIBA-GEIGY)<br>* Beispiele 1-2; Anspruch 3 *<br>– – – – – | 1-5 | |

| RECHERCHIERTE SACHGEBIETE (Int. Cl.5) |
|---|
| C 07 C 309/00 |

Der vorliegende Recherchenbericht wurde für alle Patentansprüche erstellt

| Recherchenort | Abschlußdatum der Recherche | Prüfer |
|---|---|---|
| Den Haag | 24 September 91 | ZERVAS B. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument